# FASCICULE DE BREVET EUROPEEN

(11) **EP 1 547 538 B1**
(45) Date de publication et mention de la délivrance du brevet: **06.09.2006**
(21) Numéro de dépôt: 04293139.4
(22) Date de dépôt: 27.12.2004
(51) Int. Cl.: A61B 18/20

(54) **Pièce à main comportant au moins un tube flash**
Handstück mit zumindest einer Blitzlampe
Hand piece including at least one flash lamp

(30) Priorité: 26.12.2003 FR 0315455
(43) Date de publication de la demande: 29.06.2005
(73) Titulaire: Eurofeedback, 91017 Evry Cedex (FR)
(72) Inventeur: Safraoui, Georges, 91140 Villejust (FR)
(74) Mandataire: Tanty, François

(56) Documents cités:
- EP-A- 1 232 767
- WO-A-99/32193
- FR-A- 2 838 954

## Description

La présente invention concerne les appareils pour effectuer des traitements esthétiques ou médicaux par émission de flashs lumineux, et plus particulièrement les pièces à main utilisées dans de tels appareils.

La demande de brevet français FR 2 838 954 décrit une pièce à main comportant une tête optique comprenant un corps en matière plastique présentant une cavité traversée par un tube flash. Une circulation d'eau est organisée dans la cavité pour refroidir le tube.

D'autres têtes optiques connues comportent un corps métallique, ce qui accroît le poids de la pièce à main.

Les têtes optiques dont le corps est en matière plastique présentent parfois une durée de vie réduite, due à une dégradation inattendue de la matière plastique sous l'effet de la lumière émise par le tube flash, notamment dans les régions d'extrémité de la cavité qui sont pourvues des ouvertures permettant le passage du tube flash.

Cette dégradation peut s'expliquer de la manière suivante.

La matière plastique comporte quelquefois des impuretés microscopiques qui absorbent le rayonnement provenant du tube flash, s'échauffent puis carbonisent à la longue, ce qui augmente encore leur pouvoir d'absorption de la lumière, et entraîne progressivement la dégradation de la matière plastique environnante, qui finit par carboniser à son tour. Cela peut entraîner une contamination du liquide de refroidissement qui cesse d'être parfaitement transparent et des fuites. Le document EP 1 232 767 décrit une autre pièce à main selon la préambule de la revendication 1.

Les pièces à main dont la tête optique comporte un corps en matière plastique doivent donc être testées après leur fabrication, pour éliminer celles dont la matière plastique se dégrade rapidement en raison de la présence d'impuretés, comme expliqué ci-dessus, ce qui se répercute sur le prix de revient.

Il existe un besoin pour améliorer encore les pièces à main connues et notamment accroître la fiabilité de la tête optique ainsi que la durée de vie du tube flash.

L'invention vise notamment à répondre à ce besoin.

Elle y parvient, selon l'un de ses aspects, en proposant une pièce à main, comportant au moins un tube flash et un corps en matière plastique comportant une cavité traversée par le tube flash à la faveur d'ouvertures réalisées dans des régions d'extrémité de la cavité, cette pièce à main pouvant se caractériser par le fait qu'elle comporte au moins un écran protégeant au moins partiellement au moins une région d'extrémité de la lumière provenant du tube flash.

Ainsi, les régions d'extrémité peuvent recevoir moins d'énergie et le risque de dégradation de la matière plastique est réduit.

La tête optique comporte avantageusement un réflecteur pour réfléchir la lumière émise par le tube flash dans une direction sensiblement perpendiculaire à l'axe longitudinal du tube flash. Ce réflecteur présente avantageusement une surface diffusante, étant réalisé par exemple dans une céramique.

L'écran précité peut être réalisé de diverses façons.

L'écran peut notamment être constitué au moins partiellement par une paroi d'une pièce métallique rapportée sur le corps en matière plastique.

Dans un exemple de réalisation, la pièce à main comporte un bouclier thermique s'étendant au moins partiellement entre le réflecteur et le corps en matière plastique. L'écran peut être constitué au moins partiellement par une portion de ce bouclier.

L'écran peut encore être réalisé, en variante, au moins partiellement par une paroi du réflecteur qui s'étend perpendiculairement à l'axe longitudinal du tube flash.

L'écran peut encore être constitué au moins partiellement par une métallisation de la cavité ou par le dépôt d'un revêtement réfléchissant sur celle-ci.

Le bouclier précité est avantageusement métallique et comporte au moins une languette élastique en contact électrique avec un conducteur d'amenée d'une tension de déclenchement d'un éclair dans le tube flash. Ce conducteur peut comporter par exemple une vis dont la tête est serrée contre le fond de la cavité du corps en matière plastique, ce qui permet d'obtenir une traversée étanche du corps en matière plastique sans avoir à utiliser de joint d'étanchéité.

Le corps en matière plastique de la tête optique comporte avantageusement des canaux d'amenée et de retour d'un liquide de refroidissement du tube flash.

Dans un exemple de réalisation, l'un au moins des canaux d'amenée et de retour est orienté de manière à générer un écoulement hélicoïdal du liquide de refroidissement autour du tube flash. Cela permet d'obtenir un refroidissement plus homogène du tube flash et peut accroître sa durée de vie.

De préférence, un joint d'étanchéité s'applique sur le tube flash à chaque extrémité de celui-ci, et chaque joint d'étanchéité se situe en arrière de l'électrode correspondante. Un tel positionnement du joint d'étanchéité permet d'augmenter la surface de l'enveloppe du tube flash au contact du liquide de refroidissement et d'améliorer encore la qualité du refroidissement du tube flash.

Le joint d'étanchéité précité est par exemple logé dans une gorge annulaire radialement intérieure d'une bague rapportée sur le corps en matière plastique, cette bague comportant par exemple une gorge annulaire radialement extérieure se superposant avec la gorge annulaire radialement intérieure et logeant un deuxième joint d'étanchéité. Le bouchon formé par la bague et les deux joints d'étanchéité peut présenter un encombrement axial relativement faible, ce qui permet au premier joint d'étanchéité de s'appliquer sur le tube flash à une distance relativement faible de l'extrémité métallique de celui-ci, au profit de la surface d'échange thermique entre l'enveloppe du tube flash et le liquide de refroidissement.

Dans un exemple de réalisation, l'écran s'étend sur une révolution complète autour de l'axe longitudinal du tube flash, autour d'au moins une ouverture correspondante du corps en matière plastique. Lorsque l'écran est métallique, cela peut contribuer à faciliter l'allumage du tube flash et permettre ainsi d'utiliser par exemple, toutes choses égales par ailleurs, un tube flash ayant une pression de gaz supérieure, ce qui peut améliorer le rendement du tube.

Dans un exemple de réalisation, la pièce à main comporte des connecteurs engagés sur les extrémités métalliques du tube flash, ces connecteurs comportant des pinces ayant des branches surdimensionnées, verrouillées avec des agrafes.

La pièce à main peut comporter, dans des exemples de réalisation, plus d'un tube flash, par exemple au moins deux tubes flash, ce qui peut permettre d'accroître la surface traitée à chaque émission d'un flash lumineux et/ou d'exposer la surface à traiter avec un facteur de forme proche de l'unité. L'expression « facteur de forme » désigne le rapport de la plus grande dimension à la plus petite dimension. Dans le cas d'un carré, le facteur de forme est égal à un.

Dans un exemple de réalisation, l'écran comporte deux parties tubulaires, notamment deux bagues, disposées de manière à protéger de la lumière provenant du tube flash les ouvertures du corps traversées par le tube flash.

Le corps peut alors, par exemple, être réalisé en PET. La présente demande décrit encore, indépendamment ou en combinaison avec ce qui précède, une pièce à main comportant un corps comportant une cavité traversée par au moins un tube flash, ce corps comportant des canaux d'amenée et de retour d'un liquide de refroidissement dans la cavité, la pièce à main comportant en outre au moins deux joints d'étanchéité s'appliquant sur le tube, la pièce à main pouvant se caractériser par le fait que les joints d'étanchéité s'appliquent sur le tube en arrière des électrodes du tube flash.

Un tel positionnement permet d'améliorer le refroidissement du tube flash, comme expliqué plus haut.

La présente demande décrit encore indépendamment ou en combinaison avec ce qui précède, une pièce à main comportant un corps en matière plastique comportant une cavité traversée par au moins un tube flash. Ce corps peut être traversé par une vis dont la tête est serrée contre le fond de la cavité. La vis peut être reliée à un conducteur d'amenée d'une tension de déclenchement du tube flash. Une pièce métallique comportant une languette élastique peut être disposée dans la cavité, entre un réflecteur et le fond de la cavité. Cette pièce métallique peut constituer un bouclier thermique. La languette peut venir au contact d'un conducteur électrique d'amenée d'une tension de déclenchement dans le tube flash. La languette peut ainsi venir au contact de la vis précitée. Cela peut simplifier la fabrication de la pièce à main. Le bouclier thermique peut comporter des parois d'extrémité traversées par des ouvertures pour le passage du tube flash. Ces parois d'extrémité peuvent faciliter l'allumage du tube flash, tout en protégeant le corps en matière plastique de la lumière émise par le tube flash.

La présente demande décrit encore, indépendamment ou en combinaison avec ce qui précède, une pièce à main comportant un corps présentant une cavité traversée par un tube flash, la pièce à main pouvant se caractériser par le fait que le tube flash comporte des extrémités métalliques reliées à des contacteurs électriques, ces derniers comportant des pinces surdimensionnées, avantageusement verrouillées par des agrafes.

La présente demande décrit encore de ses aspects, indépendamment ou en combinaison avec ce qui précède, une pièce à main comportant un corps en matière plastique présentant une cavité traversée par au moins deux tubes flash disposés côte à côte. Ces tubes flash peuvent être reliés électriquement en série.

L'invention pourra être mieux comprise à la lecture de la description détaillée qui va suivre, d'exemples de mise en oeuvre non limitatifs de celle-ci, et à l'examen du dessin annexé, sur lequel :
- la figure 1 représente de manière schématique une pièce à main réalisée conformément à l'invention, après démontage partiel du boîtier,
- la figure 2 représente isolément une partie de la tête optique de la figure 1,
- la figure 3 est une vue éclatée et partielle de la tête optique de la figure 1,
- la figure 4 représente en perspective le corps en matière plastique de la tête optique,
- la figure 5 est une vue de côté selon la flèche V de la figure 2,
- la figure 6 est une coupe longitudinale, dans un plan médian, du corps en matière plastique de la tête,
- la figure 7 est une vue de dessus selon VII de la figure 6,
- la figure 8 représente isolément le bouclier,
- la figure 9 illustre le positionnement du tube relativement à l'un des bouchons,
- la figure 10 est une coupe axiale d'un bouchon,
- la figure 11 représente un détail de la figure 2, le tube flash étant enlevé,
- la figure 12 représente à échelle agrandie un connecteur fixé sur une extrémité métallique du tube flash,
- les figures 13 à 15 représentent des variantes de réalisation d'écrans,
- la figure 16 représente une variante de réalisation du corps en matière plastique de la tête optique, et
- la figure 17 représente une autre variante de réalisation de l'écran.

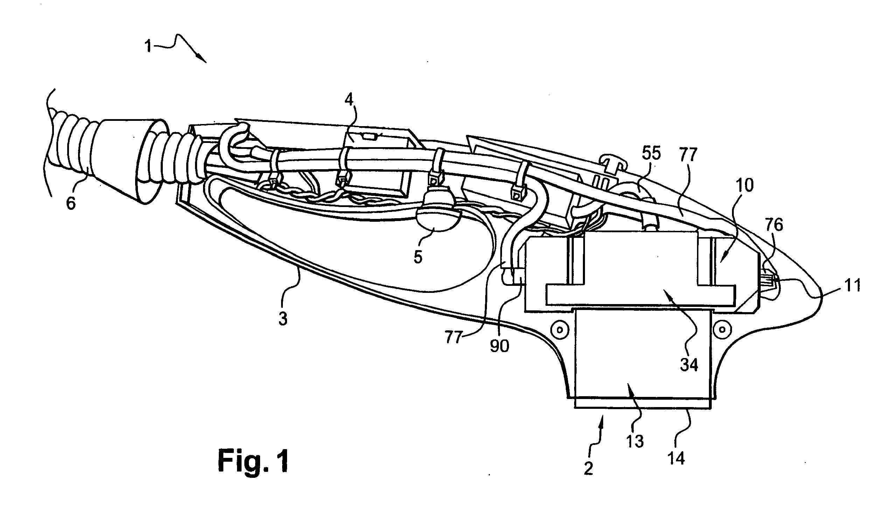

La pièce à main 1 représentée à la figure 1 comporte une tête optique 2 disposée dans un boîtier 3 composé de deux demi-coques dont seule l'une d'entre elles est apparente sur la figure 1. Le boîtier 3 loge, outre la tête optique 2, des circuits électroniques 4 et un bouton-poussoir 5 qui permet de déclencher l'émission d'un flash lumineux.

La pièce à main 1 est raccordée par un flexible 6 à un générateur électrique non représenté et correspondant par exemple à celui décrit dans la demande de brevet français de la demanderesse FR 2 838 042, dont le contenu est incorporé à la présente par référence.

La tête optique 2 comporte un corps 10, en matière plastique, de préférence blanche, par exemple du polytétrafluoroéthylène usiné ou du polyamide moulé, qui est traversé par un tube flash 11 et un guide optique 13 qui permet d'acheminer la lumière émise par le tube flash 11 vers une face de sortie 14 destinée à être appliquée contre la peau.

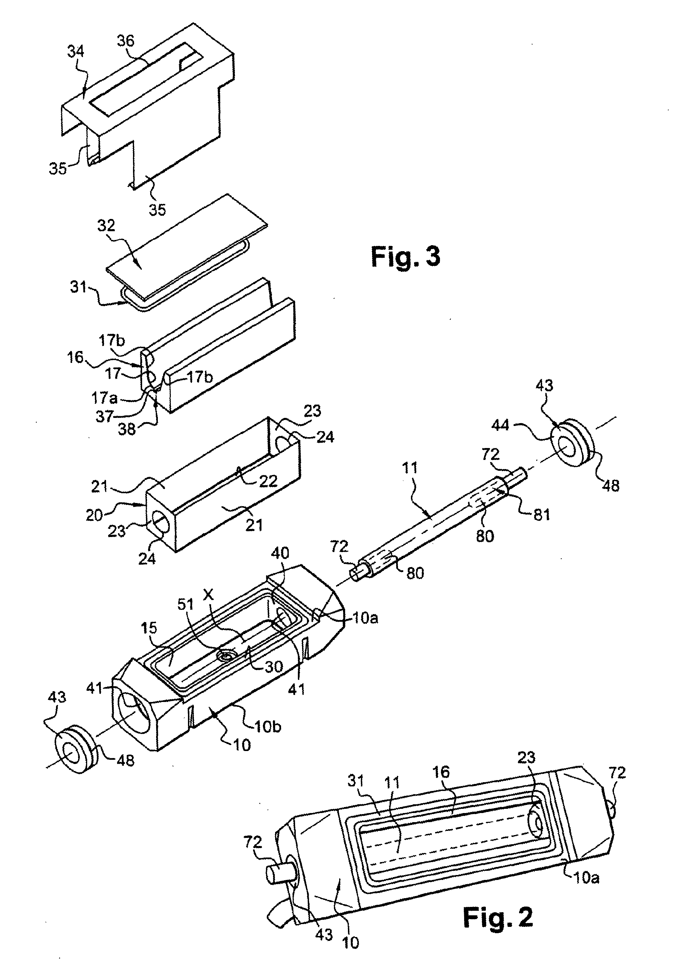

En se reportant aux figures 2 à 7, on peut voir que le corps 10 comporte une cavité 15 de forme générale parallélépipédique, allongée selon un axe longitudinal X, qui reçoit un réflecteur 16 et un bouclier thermique 20, qui s'interpose entre le réflecteur 16 et le corps 10.

Le réflecteur 16 présente une surface intérieure réfléchissante 17. Il est de préférence réalisé dans une matière présentant des propriétés de diffusion de la lumière, par exemple une céramique blanche.

La surface réfléchissante 17 peut présenter des formes diverses sans que l'on sorte du cadre de la présente invention. Dans l'exemple considéré, le réflecteur 16 présente un fond 17a de section transversale sensiblement parabolique et des flancs 17b sensiblement plans.

Le bouclier 20 est réalisé, dans l'exemple considéré, par découpage et pliage d'une tôle, par exemple de l'acier inoxydable à ressort, et présente deux parois longitudinales 21, une paroi de fond 22 et deux parois d'extrémité 23, traversées chacune par une ouverture 24 permettant le passage du tube flash 11. Les ouvertures 24 font par exemple 9 mm de diamètre, celui du tube 11 étant par exemple de 6 mm environ à leur niveau.

Le corps 10 présente, sur sa face 10a tournée du côté du guide optique 13, une gorge 30 pour accueillir un joint d'étanchéité 31 contre lequel est pressé un filtre 32 par exemple de couleur rouge, lequel est lui-même maintenu comprimé contre le joint 31 par une pièce de maintien 34 comportant deux montants 35 encliquetés sur des reliefs prévus à cet effet sur la face 10b du corps 10 située à l'opposé du guide optique 13. Le filtre 32 peut être choisi en fonction de l'utilisation, par exemple de la couleur des poils à éliminer ou de la nature du traitement, notamment épilation ou photo-rajeunissement ou autre.

La pièce de maintien 34 comporte une ouverture 36 dont la section correspond sensiblement à celle du guide optique 13 et dans laquelle celui-ci est engagé à une extrémité.

On peut voir également sur la figure 3 que le réflecteur 16 comporte une gorge longitudinale 37 dans laquelle est engagé un fil conducteur 38, qui fait le tour du réflecteur 16 dans le sens de sa longueur, qui est en contact avec le bouclier 20 et qui facilite l'allumage du tube flash 11.

La cavité 15 est délimitée axialement par des régions d'extrémité 40 qui s'étendent généralement perpendiculairement à l'axe X et dans lesquelles débouchent des ouvertures 41 destinées au passage du tube 11. Ces ouvertures 41 débouchent à l'extérieur du corps 10 par des lamages 42 permettant d'accueillir des bouchons 43.

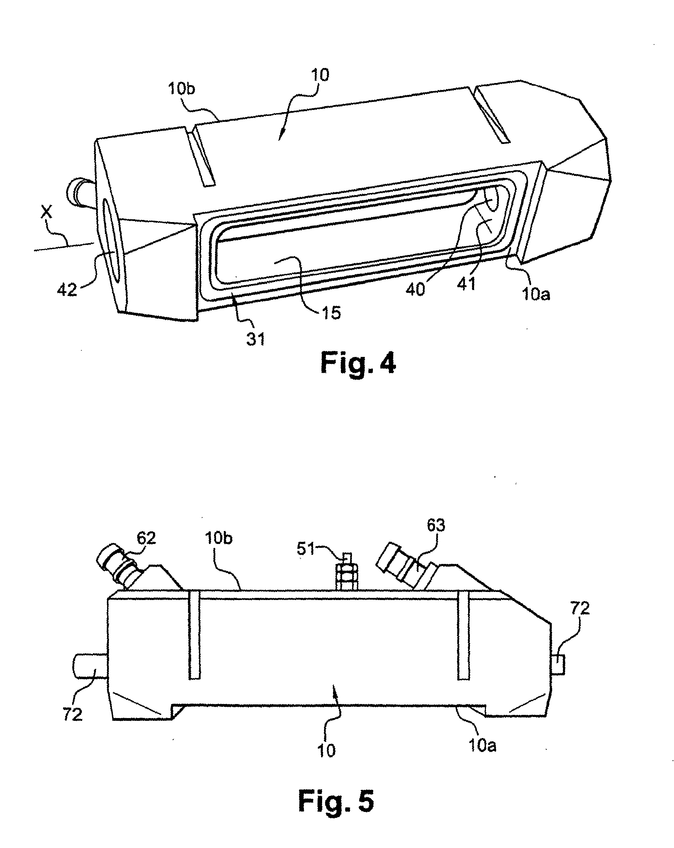

Le corps 10 est traversé par un trou 50 qui débouche dans le fond de la cavité 15 et qui permet la fixation d'une vis 51, comme on le voit sur les figures 3 et 5. La tête de cette vis 51 vient en appui dans le fond d'un lamage 53 et obture de manière étanche le trou 50.

La vis 51 est reliée à un conducteur électrique 53 d'amenée d'une tension d'allumage du tube.

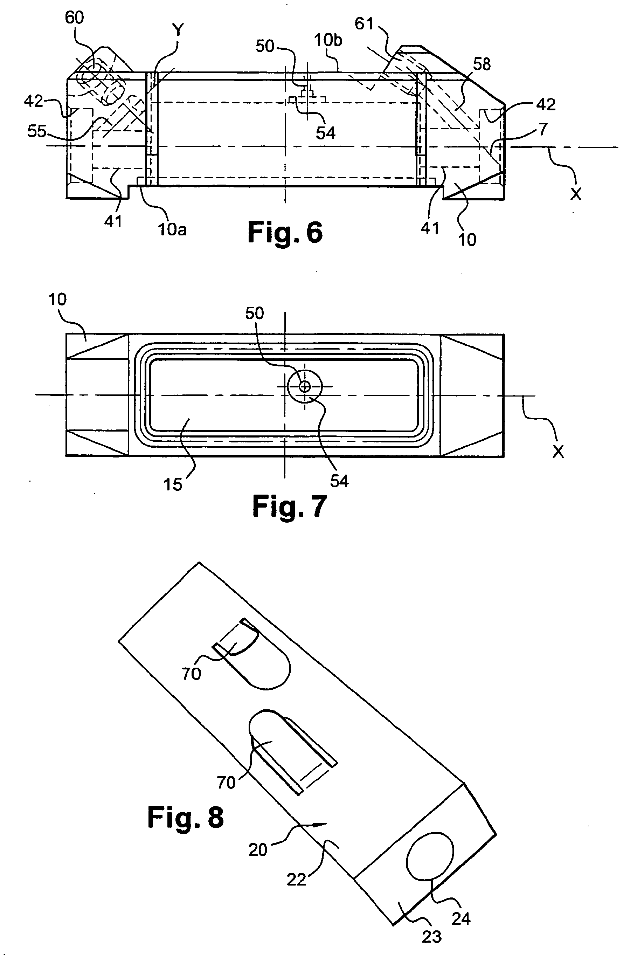

Le bouclier 20 a été représenté isolément à la figure 8. On peut voir sur celle-ci que sa paroi de fond 22 comporte deux languettes 70 qui présentent une certaine élasticité et qui sont disposées de telle sorte que lorsque le bouclier 20 est en place dans la cavité 15, l'une des languettes 70 s'applique contre la tête de la vis 51, ce qui établit un contact électrique entre le bouclier 20 et le conducteur 53 d'amenée de la tension de déclenchement.

Pour refroidir le tube flash 11, une circulation d'un liquide de refroidissement, en l'espèce de l'eau déminéralisée, est organisée dans la cavité 15.

Le liquide de refroidissement est amené par des canaux intérieurs 55 et 56, comme on le voit sur la figure 6, qui débouchent à proximité du fond du lamage 42, et dont les axes respectifs Y et Z sont orientés obliquement relativement au plan de coupe de la figure 6, de manière à générer autour du tube flash 11 dans la cavité 15 un écoulement hélicoïdal.

Les canaux 55 et 56 communiquent avec des logements respectifs 60 et 61 qui servent à la fixation sur le corps 10 d'embouts 62 et 63 de raccordement à des tubulures d'amenée et de départ du liquide de refroidissement.

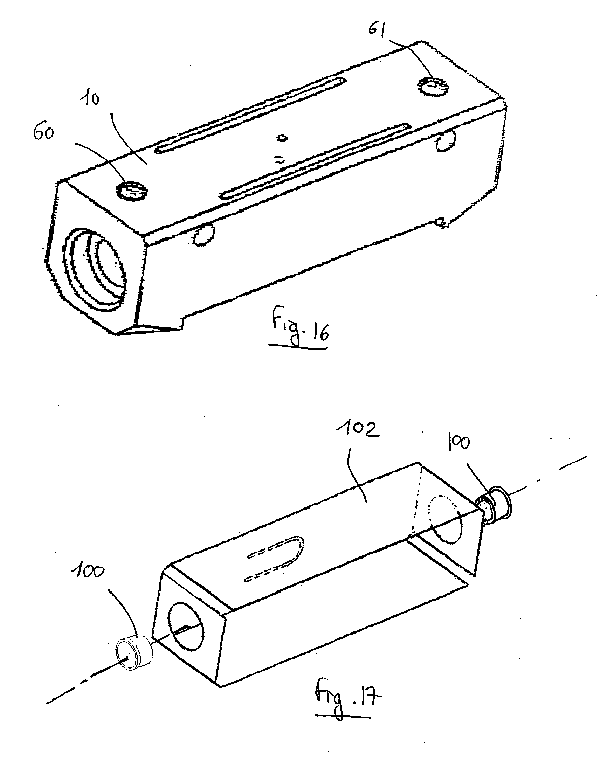

Dans la variante de réalisation de la figure 16 les logements 60 et 61 sont réalisés différemment, étant orientés sensiblement perpendiculairement à l'axe longitudinal du corps.

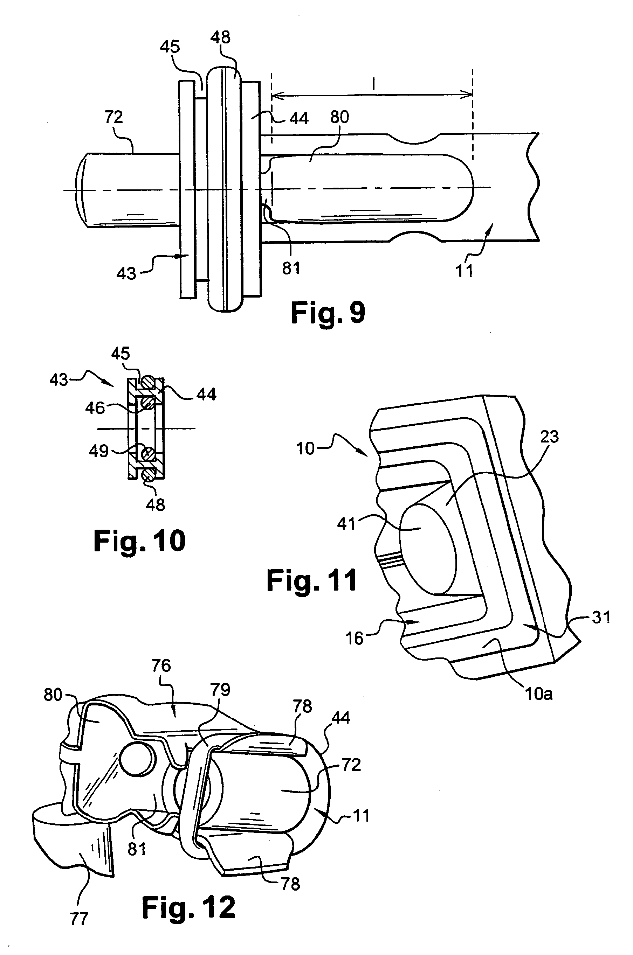

Chaque bouchon 43 comporte, comme on peut le voir sur la figure 10, une bague 44 et deux joints d'étanchéité toriques 48 et 49.

Le joint d'étanchéité 48 est reçu dans une gorge annulaire 45, radialement extérieure, de la bague 44, tandis que le joint d'étanchéité 49 est reçu dans une gorge annulaire 46, radialement intérieure, de la bague 44, les gorges 45 et 46 se superposant.

Le joint d'étanchéité 48 s'applique sur la périphérie du lamage 42 tandis que le joint d'étanchéité 49 s'applique sur le tube flash 11.

Le tube flash 11 comporte des extrémités métalliques 72 qui se raccordent par des conducteurs internes 81 à des électrodes 80, comme on peut le voir sur la figure 9.

Le positionnement de chaque bouchon 43 relativement au tube flash 11 permet au liquide de refroidissement de circuler tout autour de l'électrode correspondante 80 et sur toute la longueur *l* de celle-ci.

Lorsque le réflecteur 16 est en place dans le bouclier 20, les régions d'extrémité 40 de la cavité 15 sont protégées vis-à-vis du rayonnement lumineux par les parois d'extrémité 23 du bouclier 20, comme on peut le voir sur la figure 11.

Les extrémités métalliques 72 dépassent hors du corps 10 et supportent des contacteurs 76, lesquels comportent des pinces sur lesquelles sont soudés des câbles 77 d'alimentation électrique du tube flash 11.

On peut voir sur la figure 12 que chaque pince 76 présente des branches 78 surdimensionnées et maintenues serrées contre l'extrémité métallique 72 correspondante du tube flash 11 par une agrafe 79.

Les branches 78 sont reliées entre elles par une paroi 90 qui est relativement éloignée de l'extrémité métallique 72, de manière à ménager un espace 81 dans lequel l'air peut circuler et dont le volume est au moins égal, sinon du même ordre, que celui de l'extrémité métallique 72.

Bien entendu, l'invention n'est pas limitée à l'exemple de réalisation qui vient d'être décrit.

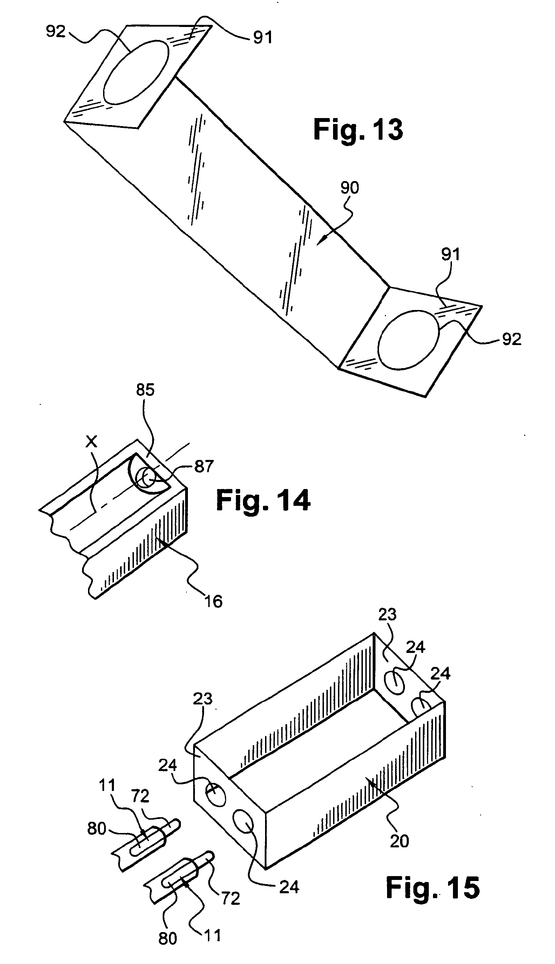

En particulier, on peut réaliser l'écran qui réduit la quantité de lumière atteignant les régions d'extrémité 40 de la cavité 15 différemment, et par exemple modifier la forme du réflecteur 16 précédemment décrit de manière à réaliser celui-ci comme illustré sur la figure 14 avec des parois d'extrémité 85 s'étendant perpendiculairement à l'axe longitudinal X, ces parois d'extrémité 85 étant traversées par des ouvertures 87 pour le passage du tube flash 11.

Dans une autre variante, la cavité 15 reçoit au moins par endroits et notamment sur ses régions d'extrémité 40 un revêtement réfléchissant, par exemple sous forme d'une métallisation ou du dépôt d'une peinture réfléchissante.

Il convient de noter qu'il n'est pas nécessaire que l'écran intercepte toute la lumière provenant du tube flash 11. Il suffit qu'il en intercepte suffisamment pour que le risque de dégradation de la matière plastique soit diminué dans la mesure désirée.

L'invention n'est pas limitée à une tête optique comportant un seul tube flash et elle s'applique également à une tête optique comportant au moins deux tubes flash 11, comme illustré à la figure 15. Ces tubes flash 11 peuvent s'étendre parallèlement l'un à l'autre et être reliés électriquement en série.

Le bouclier 20 peut alors être réalisé avec des parois d'extrémité 23 traversée chacune par deux orifices 24 pour le passage des tubes flash 11, mais d'autres écrans peuvent être utilisés, tout ce qui vient d'être décrit en référence à un seul tube flash 11 demeurant valable lorsque plusieurs tubes flash sont utilisés.

L'écran destiné à protéger les régions d'extrémité 40 de la cavité 15 peut encore être réalisé sous la forme d'une pièce métallique distincte du bouclier 20, par exemple sous la forme de celle représentée à la figure 13, comportant une paroi de fond 90 destinée à venir au contact du bouclier 20, sous le réflecteur 16, et deux parois d'extrémité 91 traversées chacune par une ouverture 92 permettant le passage du tube flash 11. Le bouclier 20 peut alors, le cas échéant, être réalisé sans les parois d'extrémité 23.

On a représenté à la figure 17 un écran 102 selon une variante de mise en oeuvre de l'invention.

Cet écran peut comporter, comme illustré à la figure 17, des bagues 100 destinées à protéger la surface intérieure des ouvertures 41 du corps 10 du rayonnement lumineux produit par le tube flash engagé dans celles-ci.

La présence des bagues 100 et la meilleure protection qui en résulte pour le corps 10 peut permettre d'utiliser, pour réaliser celui-ci, une matière plastique plus facile à usiner que le PTFE, par exemple du PET, notamment celui commercialisé sous la dénomination commerciale TECADOR® par la société britannique ENSINGER.

Dans toute la description, y compris les revendications, l'expression « comportant un » doit être comprise comme étant synonyme de « comportant au moins un », sauf si le contraire est spécifié.

## Revendications

1. Pièce à main (1) pour réaliser un traitement par émission de flashs lumineux, notamment pour l'épilation, comportant :
- au moins un tube flash (11),
- un corps (10) en matière plastique, comportant une cavité (15) traversée par le tube flash (11) à la faveur d'ouvertures (41) réalisées dans des régions d'extrémité (40) de la cavité (15), au moins un écran (23 ; 85 ; 91 ; 100, 102) protégeant au moins partiellement au moins une de ces régions d'extrémité (40) de la lumière provenant du tube flash (11).

2. Pièce selon la revendication 1, **caractérisée par le fait que** le corps (10) comporte des canaux (55, 56) d'amenée et de retour d'un liquide de refroidissement du tube flash (11), et **par le fait que** l'un au moins du canal d'amenée et de retour est orienté de manière à générer un écoulement hélicoïdal du liquide de refroidissement autour du tube flash (11).

3. Pièce selon la revendication 1 ou 2, **caractérisée par le fait qu'**un joint d'étanchéité (49) s'applique sur le tube (11) à chaque extrémité de celui-ci, en arrière d'une électrode correspondante (80).

4. Pièce selon la revendication 3, **caractérisée par le fait que** le joint d'étanchéité (49) est logé dans une gorge annulaire radialement intérieure (46) d'une bague (44) rapportée sur le corps (10), cette bague comportant une gorge annulaire radialement extérieure (45) se superposant à la gorge annulaire radialement intérieure (46).

5. Pièce selon l'une quelconque des revendications 1 à 4, **caractérisée par le fait que** l'écran (23 ; 85 ; 91) s'étend sur une révolution complète autour de l'axe longitudinal du tube flash, autour de chaque ouverture (41) du corps (10).

6. Pièce selon l'une quelconque des revendications 1 à 5, **caractérisée par le fait qu'**elle comporte un réflecteur (16) pour réfléchir la lumière émise par le tube flash (11) dans une direction sensiblement perpendiculaire à l'axe longitudinal du tube flash, et **par le fait que** le réflecteur (16) présente de préférence une surface réfléchissante (17) agencée pour diffuser la lumière.

7. Pièce selon l'une quelconque des revendications précédentes, **caractérisée par le fait que** l'écran (23 ; 91) est constitué au moins partiellement par une paroi d'une pièce métallique (20 ; 90) rapportée sur le corps (10) en matière plastique.

8. Pièce selon l'une quelconque des revendications 1 à 6, **caractérisée par le fait que** l'écran comporte un dépôt d'un métal ou d'un revêtement réfléchissant.

9. Pièce selon la revendication 6, **caractérisée par le fait que** l'écran est constitué au moins partiellement par une paroi d'extrémité (85) du réflecteur (16).

10. Pièce selon la revendication 6, **caractérisée par le fait qu'**elle comporte un bouclier (20) s'étendant au moins partiellement entre le réflecteur (16) et le corps (10) en matière plastique, de préférence un bouclier métallique.

11. Pièce selon la revendication 10, **caractérisée par le fait que** l'écran (23) est constitué au moins partiellement par une portion du bouclier (20).

12. Pièce selon la revendication 10, **caractérisée par le fait que** le bouclier (20) comporte au moins une languette élastique (70) en contact électrique avec un conducteur (51) d'amenée d'une tension de déclenchement d'un éclair dans le tube flash (11).

13. Pièce selon la revendication 12, **caractérisée par le fait que** le conducteur comporte une vis (51) dont la tête est serrée contre le fond de la cavité (15).

14. Pièce selon l'une quelconque des revendications précédentes, **caractérisée par le fait qu'**elle comporte des contacteurs (76) engagés sur des extrémités métalliques (72) du tube flash (11), ces contacteurs comportant des pinces dont les branches (78) sont surdimensionnées et verrouillées avec des agrafes (79).

15. Pièce selon l'une quelconque des revendications précédentes, **caractérisée par le fait qu'**elle comporte deux tubes flash (11).

16. Pièce selon l'une quelconque des revendications précédentes, **caractérisée par le fait que** l'écran (102) comporte deux partie tubulaires, notamment deux bagues (100), disposées de manière à protéger de la lumière provenant du tube flash les ouvertures (41) du corps (10) traversées par le tube flash.

17. Pièce selon la revendication 16, **caractérisée par le fait que** le corps (10) est en PET.

## Claims

1. Hand piece (1) for performance of a treatment by emission of light flashes, in particular for depilation, comprising:
- at least one flash tube (11),
- a body (10) of plastics material comprising a cavity (15) through which passes the flash tube (11) via openings (41) made in the end regions (40) of the cavity (15),
- at least one screen (23; 85; 91; 100; 102) at least partly protecting at least one of these end regions (40) from the light emitted by the flash tube (11).

2. Piece according to claim 1, **characterised in that** the body (10) comprises channels (55, 56) for supply and return of a cooling liquid for the flash tube (11) and **in that** at least one of the supply and return channels is oriented so as to generate a helicoidal flow of the cooling liquid around the flash tube (11).

3. Piece according to claim 1 or 2, **characterised in that** a sealing joint (49) is applied to the tube (11) at each end behind a corresponding electrode (80).

4. Piece according to claim 3, **characterised in that** the sealing joint (49) is housed in an radially inner annular groove (46) of a ring (44) mounted on the body (10), this ring comprising a radially outer annular groove (45) superimposed on the radially inner annular groove (46).

5. Piece according to any of claims 1 to 4, **characterised in that** the screen (23; 85; 91) extends over a complete revolution about the longitudinal axis of the flash tube around each opening (41) in the body (10).

6. Piece according to any of claims 1 to 5, **characterised in that** it comprises a reflector (16) to reflect the light emitted by the flash tube (11) in a direction approximately perpendicular to the longitudinal axis of the flash tube and **in that** the reflector (16) preferably has a reflective surface (17) arranged to diffuse the light.

7. Piece according to any of the previous claims, **characterised in that** the screen (23; 91) comprises at least partly a wall of a metal piece (20; 90) mounted on the body (10) of plastics material.

8. Piece according to any of claims 1 to 6, **characterised in that** the screen comprises a deposit of a metal or a reflective coating.

9. Piece according to claim 6, **characterised in that** the screen comprises at least partly an end wall (85) of the reflector (16).

10. Piece according to claim 6, **characterised in that** it comprises a shield (20) extending at least partly between the reflector (16) and the body (10) of plastics material, preferably a metal shield.

11. Piece according to claim 10, **characterised in that** the screen (23) is at least partly constituted by a portion of the shield (20).

12. Piece according to claim 10, **characterised in that** the shield (20) comprises at least one resilient tab (70) in electrical contact with a conductor (51) supplying a voltage to trigger a flash in the flash tube (11).

13. Piece according to claim 12, **characterised in that** the conductor comprises a screw (51), the head of which is clamped against the base of the cavity (15).

14. Piece according to any of the preceding claims, **characterised in that** it comprises contactors (76) engaged on the metallic ends (72) of the flash tube (11), these contactors comprising pincers, the arms (78) of which are over-dimensioned and screwed with clips (79).

15. Piece according to any of the previous claims, **characterised in that** it comprises two flash tubes (11).

16. Piece according to any of the preceding claims, **characterised in that** the screen (102) comprises two tubular parts in particular two rings (100), arranged to protect the openings (41) of the body (10), through which the flash tube passes, from the light emitted by the flash tube.

17. Piece according to claim 16, **characterised in that** the body (10) is made of PET.

## Patentansprüche

1. Handstück für eine Blitzlichtbehandlung, insbesondere zur Epilation, mit:
- mindestens einer Blitzröhre (11),
- einem Körper (10) aus Kunststoff, der eine Höhlung (15) aufweist, die von der Blitzröhre (11) in Öffnungen (41) durchquert wird, die in Endbereichen (40) der Höhlung (15) ausgebildet sind,
- wenigstens einem Schirm (23; 85; 91; 100; 102), der zumindest einen dieser Endbereiche (40) zumindest teilweise gegen das von der Blitzröhre (11) kommende Licht schützt.

2. Handstück nach Anspruch 1, **dadurch gekennzeichnet, daß** der Körper (10) Kanäle (55, 56) zur Zuleitung und Rückleitung einer Kühlflüssigkeit für die Bitzröhre (11) aufweist und daß wenigstens einer der Zuführungs- und Rückleitungskanäle so orientiert ist, daß er eine schraubenförmige Strömung der Kühlflüssigkeit um die Blitzröhre (11) herum erzeugt.

3. Handstück nach Anspruch 1 oder 2, daß an jedem Ende der Blitzröhre (11) hinter einer entsprechenden Elektrode (80) eine Dichtung (49) angebracht ist.

4. Handstück nach Anspruch 3, **dadurch gekennzeichnet, daß** die Dichtung (49) in einer radial nach innen gerichteten Ringnut (46) eines an den Körper (10) angesetzten Ringes (44) aufgenommen ist und daß dieser Ring eine radial nach außen gerichtete Ringnut (45) aufweist, die der radial nach innen gerichteten Ringnut (46) überlagert ist.

5. Handstück nach einem der Ansprüche 1 bis 4, **dadurch gekennzeichnet, daß** der Schirm (23; 85; 91) sich um jede Öffnung (41) des Körpers (10) herum in einer vollständigen Windung um die Längsachse der Blitzröhre erstreckt.

6. Handstück nach einem der Ansprüche 1 bis 5, **dadurch gekennzeichnet, daß** es einen Reflektor (16) zum Reflektieren des von der Blitzröhre (11) emittierten Lichts in eine zu der Längsachse der Blitzröhre im wesentlichen senkrechte Richtung aufweist und daß der Reflektor (16) vorzugsweise eine reflektierende Oberfläche (17) bildet, die dazu ausgebildet ist, das Licht zu streuen.

7. Handstück nach einem der vorstehenden Ansprüche, **dadurch gekennzeichnet, daß** der Schirm (23; 191) zumindest zum Teil durch eine Wand eines Metallteils (20; 90) gebildet wird, das an dem Körper (10) aus Kunststoff angebracht ist.

8. Handstück nach einem der Ansprüche 1 bis 6, **dadurch gekennzeichnet, daß** der Schirm eine Metallisierung oder eine reflektierende Verkleidung aufweist.

9. Handstück nach Anspruch 6, **dadurch gekennzeichnet, daß** der Schirm zumindest zum Teil durch eine Stirnwand (85) des Reflektors (16) gebildet wird.

10. Handstück nach Anspruch 6, **dadurch gekennzeichnet, daß** es einen Schutzschild (20), vorzugsweise aus Metall, aufweist, der sich zumindest zum Teil zwischen dem Reflektor (16) und dem Körper (10) aus Kunststoff erstreckt.

11. Handstück nach Anspruch 10, **dadurch gekennzeichnet, daß** der Schirm (23) zumindest zum Teil durch einen Teil des Schutzschildes (20) gebildet wird.

12. Handstück nach Anspruch 10, **dadurch gekennzeichnet, daß** der Schutzschild (20) wenigstens eine elastische Zunge (70) aufweist, die mit einem Leiter (51) zur Zufuhr einer Zündspannung für einen Blitz in der Blitzröhre (11) in elektrischem Kontakt steht.

13. Handstück nach Anspruch 12, **dadurch gekennzeichnet, daß** der Leiter eine Schraube (51) aufweist, deren Kopf gegen den Boden der Höhlung (15) gespannt ist.

14. Handstück nach einem der vorstehenden Ansprüche, **dadurch gekennzeichnet, daß** er Kontakte (76) aufweist, die an metallischen Enden (72) der Blitzröhre (11) anliegen und Zangenklemmen aufweisen, deren Schenkel (78) überdimensioniert und mit Spangen (79) verriegelt sind.

15. Handstück nach einem der vorstehenden Ansprüche, **dadurch gekennzeichnet, daß** es zwei Blitzröhren (11) aufweist.

16. Handstück nach einem der vorstehenden Ansprüche, **dadurch gekennzeichnet, daß** der Schirm (102) zwei rohrförmige Teile, insbesondere zwei Ringe (100) aufweist, die so angeordnet sind, daß sie die Öffnungen (41) des von der Blitzröhre durchsetzten Körpers (10) gegen das von der Blitzröhre kommende Licht schützen.

17. Handstück nach Anspruch 16, **dadurch gekennzeichnet, daß** der Körper (10) aus PET besteht.
